# EUROPEAN PATENT APPLICATION

(11) **EP 2 472 257 A1**
(43) Date of publication of application: **04.07.2012**
(21) Application number: 10812045.2
(22) Date of filing: 30.08.2010
(51) Int. Cl.: G01N 33/50, A61B 1/00, C12M 3/00, C12Q 1/02, C12Q 1/68

(54) **SPECIMEN FOR DETECTING INFILTRATIVE LARGE INTESTINE TUMORS**

(30) Priority: 28.08.2009 US 238106 P
(71) Applicant: Sapporo Medical University, Sapporo-shi, Hokkaido 060-0061 (JP)
(72) Inventor: TOYOTA, Minoru, Sapporo-shi Hokkaido 060-8556 (JP); YAMAMOTO, Eiichiro, Sapporo-shi Hokkaido 060-8556 (JP); KAMIMAE, Seiko, Sapporo-shi Hokkaido 060-8556 (JP); SUZUKI, Hiromu, Sapporo-shi Hokkaido 060-8556 (JP); YAMANO, Hiroo, Akita-shi Akita 010-1495 (JP)
(74) Representative: Held, Stephan
(86) International application number: PCT/JP2010/064715
(87) International publication number: WO 2011/024999

(57) **Abstract**

Provided is a method for non-invasively obtaining an index for diagnosing the infiltration or invasive depth of a large intestine tumor. The method is **characterized in that** a specimen by which the infiltrative large intestine tumor can be detected is obtained by spraying a cleaning solvent onto the target large intestine mucous layer to separate the mucous from said mucous layer, and recovering the separated mucous along with the cleaning solvent.

## Description

### [Technical Field]

The present invention relates to a specimen for detecting invasive colorectal tumors which is obtained by non-invasively detaching the colonic mucous layer, a kit for non-invasively detaching the colonic mucous layer, a method for obtaining an index to detect invasive colorectal tumors by non-invasively detaching the colonic mucous layer, and a method for obtaining an index to evaluate therapeutic effects of a drug and/or a therapeutic method by non-invasively detaching the colonic mucous layer. More specifically, the present invention relates to said specimen and kit for detecting invasive colorectal tumors and said method for obtaining an index to detect invasive colorectal tumors and saidmethod for obtaining an index to evaluate therapeutic effects, wherein methylated DNA is used as a molecular marker.

### [Background Art]

In Europe and the United States, colorectal cancer ranks high in cancer mortality. According to the prediction by statistical data in the United States in 2006, colorectal cancer is the third most commonly diagnosed cancer in both male and female, and its incidence increased annually by 1.8% from 1998 to 2002. In recent years, the number of patients with colorectal cancer has rapidly increased in Japan as well. This is considered to be because Japanese diet has changed to the European and American diet where meat is the center. In Japan, it is reported that approximately 100,000 people are annually diagnosed to have colorectal cancer, and approximately 41,000 people die. With respect to the number of death by organs, the number of colorectal cancer is the third largest following gastric cancer and lung cancer, and a further increase is being predicted. In particular, in females, colorectal cancer ranks the first among all malignant tumors, both in the number of patients diagnosed and the number of death. In males, colorectal cancer is predicted to be the third following lung cancer and liver cancer. Epidemiologically, colorectal cancer is presumed to be caused by diet, in particular by excessive intake of animal fat and protein, rather than genetic predisposition; regarding the site in the large intestine, colorectal cancer is easily developed in the sigmoid colon and the rectum.

However, different from other cancers, it is known that colorectal cancer is nearly 100% curable by surgery if detected early. Accordingly, colorectal cancer has been a subject of early cancer screening, and a number of test methods have been developed.

In addition, endoscopic surgeries such as endoscopic demucosation and endoscopic submucosal dissection are very effective for early cancer. Meanwhile, in invasive tumors, therapeutic methods such as laparotomy in combination with chemotherapeutic agents or radiation therapy, etc., are generally carried out. Therefore, development of a diagnostic method that can non-invasively evaluate invasiveness or invasion depth of cancer or tumor has been desired.

The large intestine has a five-layered structure consisting of, from the lumen side, mucosa, submucosa, muscularis propria, subserosa, and serosa; in the lower rectum, a three-layered structure excluding the serosa and subserosa is formed. Colorectal tumors originate from the mucosa, and infiltrate into deep layers as the tumors progress. Tumors in which their invasion is limited to the submucosa are called early cancer.

General test methods for digestive tract cancers, in particular colorectal cancer and rectum cancer, etc. include (i) fecal occult blood test, (ii) digital rectal examination, (iii) bloodtest, (iv) enema examination, (v) positronemission computed tomography (PET), (vi) endoscopy, (vii) capsule endoscopy, (viii) gene diagnosis using feces or biopsy sample.

However, these test methods aim at detection of early cancer, measurement of therapeutic effects, provision of materials for determining recurrence and metastasis, or definite diagnosis, and any of these methods cannot provide an index to determine the degree of invasion of cancer or tumor.

Fecal occult blood test is a method to indirectly predict occurrence of colorectal tumors by checking the presence/absence and the amount, etc. of the blood in feces, utilizing a peroxidase activity in human hemoglobin or a monoclonal antibody against human hemoglobin, and this is a simple, inexpensive and non-invasive test method. However, effectiveness of occult blood test is decreased by the fact that bleeding from a colorectal tumor is intermittent, which increases false-negative rates. For example, approximately 50% of the patients diagnosed to have a colorectal tumor are tested negative for fecal occult blood. In addition, since the amount of bleeding from small colorectal tumors with a diameter of less than 20 mm is as small as 1-2 ml per day, blood is not always detected by occult blood test. Furthermore, because positive results can be made by a number of causes other than colorectal tumors, including gingivitis, hemorrhoids, ulcers, and intestinal bleeding due to aspirin use, only 3-5% of the subjects tested positive for fecal occult blood actually have colorectal tumors, and many false-positive subjects are included in the subjects tested positive for fecal occult blood. Thus, fecal occult blood test is not a specific screening test for tumors such as colorectal tumors, and it is not necessarily sufficient as a preliminary diagnostic method of colorectal tumors. Moreover, it is impossible to determine the degree of invasion of colorectal tumors by fecal occult blood test.

By means of digital rectal examination, it is possible to detect tumors located in the far end of the rectum/colon by digital examination, but not tumors located in interior regions. Furthermore, diagnosis of the degree of invasion of colorectal tumors by digital rectal examination is not possible.

Blood tests are diagnostic method of diagnosing colorectal tumors by measuring tumor markers in the blood sample of a subject and thereby determining the amount or concentration of the tumor markers.

There are two types of tumor markers: a tumor marker, a detection of which means diagnosis of colorectal cancer, such as fetal proteins (AFP, CEA, etc.), carbohydrate antigens (CA19-9, serial Tn, etc.), and ectopically-produced substances (hormones and tumor isozyme, etc.), and a tumor marker, a detection of its genetic mutation and genetic recombination provides information, such as oncogenes (ras, erbB, etc.), tumor suppressor genes (p53, etc.), and gene rearrangement (BCR-ABL, etc.).

However, tumor markers in the blood have the following drawbacks: they may be positive even in the absence of colorectal cancer, they may not be positive until colorectal cancer grows to a certain extent, and they may not be positive even for advanced colorectal cancer. Therefoxe,tumormarkers of colorectal cancer do not show effects that lead to early detection or definite diagnosis of cancer; at present, they are used as auxiliary diagnosis and used for measurement of therapeutic effects, and as one of the criteria for detection of recurrence and metastasis. In addition, the degree of invasion of colorectal tumors cannot be determined by concentrations of tumor markers in the blood.

Enema examination is a method to examine intestinal surface irregularity by X-ray, by injecting barium into the large intestine to adhere it onto the intestinal mucosal surface. However, enema examination has problems that it is costly and subjects must bear a large burden, with a risk of complications. For example, in the enema examination, after a subject takes edema diet comprising low fat and low residue, the subject undergoes a pretreatment to eliminate the contents of the large intestine by administration of a laxative (saline purgative and contact laxative). Moreover, since enema examination only checks irregular morphology of the intestinal lumen, the degree of invasion of colorectal tumors cannot be determined by the enema examination.

Positron emission computed tomography (PET) is a method wherein a drug labeled with a positron-emitting radionuclide is administered to a subject and the quantities of the drug consumed at various sites of the body are investigated. For example, fluorodeoxyglucose labeled with 18F which is a kind of sugar and has a characteristic of accumulating in tumors is administered to a subject as a PET agent, then gamma rays are observed from external of the body, and distribution of the labeled substance inside the body is imaged to investigate its kinetics in the body, thereby determining the location and size of lesions.

PET usually requires a cyclotron and needs an expensive equipment that costs more than 1, 000, 000, 000 yen. In addition, radiation exposure cannot be avoided upon execution of PET. Moreover, although approximate size of a tumor can be measured by PET, the degree of invasion of a colorectal tumor cannot be determined.

Endoscopy is a method to investigate inside of the large intestine directly by an endoscope. Endoscopy has high sensitivity and specificity in the detection of colorectal tumors. In addition, endoscopy is advantageous in that it can excise early cancers and precancerous polyps. Furthermore, endoscopy is advantageous in that it can collect tissues for diagnosis by biopsy (tissue biopsy). However, in endoscopy, since surface of the intestine is observed from the lumen side, it is impossible to determine the degree of invasion of colorectal cancer. In fact, among tumors detected by colonoscopy, a large number of tumors that have a small lesion but invade into the submucosal tissue of the large intestine are included.

In tissue biopsy by endoscopy, only the tissue at a "point" is evaluated, and there is a limitation in extending the points to a "face." Endoscopic tissue biopsy evaluates only a part of the lesion, and therefore, definite diagnosis of colorectal cancer is impossible depending on the biopsy site, or depending on the removal site of specimens even for lesions of endoscopic mucosal ablation. Moreover, it is impossible to accurately determine the invasion depth of tumors using endoscopic tissue biopsy.

In general, a definite diagnosis of colorectal cancer is made by histopathological diagnosis using biopsy materials, so that preparation of histopathological specimens based on detailed endoscopic observation is required for definite diagnosis of colorectal tumors. However, preparation of such histopathological specimens requires a great deal of expertise, and therefore not all physicians performing endoscopy can prepare such specimens.

Regarding minute tumors and tumors before colorectal mucosectomy, biopsy makes their pathological profiles after surgery unstable, and hence, whether to perform endoscopic ablation or open abdominal surgery has been determined based only on the Endoscopic observation of size and shape as well as pit pattern diagnosis using magnifying endoscopy.

Capsule endoscopy is an examination method of gastrointestinal tracts, wherein, a patient swallaws a capsule containing a tiny camera which automatically takes pictures while it passes through the gastrointestinal tract, and the image information taken is wirelessly transmitted to outside of the body. Capsule endoscopy has the following drawbacks: the resolution of images is lower than that of general endoscopy, and since it is an automatic shooting, sufficient observation inside the folds that is the target of fine examination is impossible, and biopsy and polypectomy are impossible. Accordingly, at present capsule endoscopy is mainly used for examination of the small intestine, the observation of which is difficult by a general endoscope. Furthermore, since the intestinal surface is observed from the lumen side by capsule endoscopy as well, it is impossible to determine the degree of invasion of colorectal tumors by capsule endoscopy.

Gene diagnosis using feces or biopsy sample is a method of diagnosis of colorectal cancer by examining genes of tumor cells detached in the feces or tumor cells contained in the biopsy sample. Genetic mutation and excessive methylation of DNA occurred in tumor cells are stable information because once occurred, it is difficult to return to normal state. Therefore, when specimens are appropriate, gene diagnosis is a highly accurate diagnostic method.

However, when gene diagnosis is performed using feces, because various bacteria and nucleic acids derived from normal cells are present in the feces, the relative amount of genes derived from tumor cells collected from the feces becomes very small (approximately 0.05%), resulting in a problem that accurate diagnosis is difficult. For example, in Non-patent Literatures 1 and 2, it is described that a certain outcome has been obtained by a method of diagnosing colorectal tumors using DNA in the feces. However, these methods have problems in the possibility of diagnosis and diagnostic accuracy, and they are still far from practical application.

In contrast, when gene diagnosis is performed using biopsy samples, results vary widely depending on the collection site, which is problematic. In addition, regarding minute tumors and tumors before colorectal mucosectomy, biopsy causes fibrotic response and thermocoagulation denaturation, leading to a problem of variable pathological profiles after surgery.

Changes in genes that are the subject of gene diagnosis of colorectal tumors include mutation of carcinogenic genes such as ras, erbB, etc., mutation of tumor suppressor genes such as p53, etc., and detection of gene rearrangement such as BCR-ABL, etc., as well as epigenetic modification such as excessive methylation of CpG islands in the promoter region of tumor suppressor genes.

When a CpG island present in the promoter region of a tumor suppressor gene is methylated, transcription of this tumor suppressor gene is inactivated, leading to ineffectiveness in the control of cell growth and causing progression of cell proliferative diseases such as cancer. For example, in cancer cells, expression is inhibited by excessive methylation of CpG islands in the promoter region of genomic genes such as SFRP1, SFRP2, DKK2, hsa-mir-34b/c, p16INK4A, E-cadherin, hMLH1,14-3-3 sigma, BNIP3 that is one of BH3 Only family gene, ubiquitin ligase CHFR, CIITA that is a transcriptional coupling factor of MHC class II molecules, IGFBP7 that is a negative regulatory gene of BRAF in colorectal cancer, Histone H3K27, HRK that is an apoptosis-related gene, CACNA1G, COX2, DFNA5, and RASSF2 that is a regulatory gene of Ras; and it is reported that they are useful in diagnosis of colorectal tumors.

Excessive methylation can be detected from a minute amount of DNA, and it is stable information in that once excessive methylation occurs, it hardly returns to a normal state naturally, so that excessive methylation is considered to be useful as an index for gene diagnosis. However, while it is possible to determine the presence of tumors by gene diagnosis using feces or biopsy sample, determination of the degree of invasion of tumors is impossible.

In recent years, with advancement of endoscopic technology, the number of cases wherein colorectal tumors are cured by endoscopy without surgery has been increasing. However, there are problems such as when biopsy is performed in advance, endoscopic treatment becomes difficult, or accurate pathological diagnosis of ablation specimens becomes difficult. In some endoscopy-specialized facilities, accurate diagnosis is performed by extremely detailed magnifying endoscopic observation without biopsy, and by microscopic observation of ablation specimens; however, at present this can be performed only at limited facilities.

Thus, to date there has been no method which accurately determines genetic features of colorectal cancer cells without biopsy. In particular, there is no known method for analyzing DNA methylation of colorectal tumor cells using colonic-mucous-layer detachment fluid.

### [Citation List]

### Non-patent Literature

Non-patent Literature 1: Ahlquist DA et al., Gastroenterology. 2000 Nov; 119(5):1219-27
Non-patent Literature 2: Osborn NK et al., Gastroenterology. 2005 Jan; 128(1):192-206

### [Summary of Invention]

### Technical Problem

Therefore, an object of the present invention is to provide a specimen for detecting invasive colorectal tumors, obtained by non-invasively detach ing the colonic mucous layer, a kit for non-invasively detaching the colonic mucous layer, a method for obtaining an index to detect invasive colorectal tumors by non-invasively detaching the colonic mucous layer, and a method for obtaining an index to evaluate therapeutic effects of a drug and/or a therapeutic method by non-invasively detaching the colonic mucous layer, which do not have the above-mentioned drawbacks.

### Solution to Problem

The present inventors have devoted themselves to the research to solve the above problems, and surprisingly, they have found that it is possible to obtain a specimen for detecting invasive colorectal tumors by spraying a washing fluid onto the colonic mucous layer of a subject to detach the mucus from said mucous layer, and collecting the detached mucus together with the washing fluid; and thus the inventors have accomplished the present invention.

Namely, the present invention relates to the following specimen, kit, or method.
[1] A specimen for detecting invasive colorectal cancer, comprising a washing fluid that is obtained by spraying the washing fluid onto the colonic mucous layer to detach the colonic mucus, and collecting the detached colonic mucus together with the washing fluid.
[2] The specimen according to [1], wherein the colonic mucous layer is a colonic mucous layer that comprises a tumor site.
[3] The specimen according to [1] or [2], wherein the washing fluid is a physiological isotonic solution.
[4] The specimen according to [3], wherein the physiological isotonic solution is saline.
[5] The specimen according to any one of [1] to [4], wherein the spraying is carried out at a rate of 2-10 ml/s.
[6] The specimen according to any one of [1] to [5], wherein the detection is to detect a tumor marker.
[7] The specimen according to any one of [1] to [6], wherein the detection of a tumor marker is to detect methylated DNA.
[8] The specimen according to any one of [1] to [7], wherein the detection of methylated DNA is to detect methylated DNA in the promoter region of a gene selected from the group consisting of SFRP1, SFRP2, DKK2, hsa-mir-34b/c, p16INK4A, E-cadherin, hMLH1, 14-3-3 sigma, BNIP3, CHFR, CIITA, IGFBP7, Histone H3K27, HRK, CACNA1G, COX2, DFNA5, and RASSF2.
[9] The specimen according to [8], wherein the detection of methylated DNA is to detect methylated DNA in the promoter region of a gene selected from the group consisting of SFRP1, SFRP2, DKK2, and hsa-mir-34b/c.
[10] The specimen according to any one of [1] to [9], wherein the washing fluid is sprayed without pre-washing.
[11] The specimen according to [2], wherein the tumor site is a tumor site suspected of tumor invasion.
[12] The specimen according to any one of [1] to [11], wherein the specimen comprises 10 µg or more of DNA.
[13] A kit for detecting invasive colorectal cancer, containing a washing-fluid spraying apparatus for spraying a washing fluid onto the colonic mucous layer to detach the colonic mucus, and a washing fluid.
[14] A method for obtaining an index to detect invasive colorectal tumor, comprising:
   a step of spraying a washing fluid onto the colonic mucous layer to detach the colonic mucus,
   a step of collecting the detached colonic mucus together with the washing fluid,
   a step of determining the amount of a tumor marker in the collected washing fluid, and
   a step of obtaining an index to determine the presence of invasive colorectal tumor based on the amount of the tumor marker.
[15] The method according to [14], wherein the colonic mucous layer is a colonic mucous layer that comprises a tumor site.
[16] The method according to [14] or [15], wherein the washing fluid is a physiological isotonic solution.
[17] The method according to [16], wherein the physiological isotonic solution is saline.
[18] The method according to any one of [14] to [17], wherein the spraying is carried out at a rate of 2-10 ml/s.
[19] The method according to any one of [14] to [18], wherein the tumor marker is methylated DNA.
[20] The method according to any one of [14] to [19], wherein the tumor marker is methylated DNA in the promoter region of a gene selected from the group consisting of SFRP1, SFRP2, DKK2, hsa-mir-34b/c, p16INK4A, E-cadherin, hMLH1, 14-3-3 sigma, BNIP3, CHFR, CIITA, IGFBP7, Histone H3K27, HRK, GACNA1G, COX2, DFNA5, and RASSF2.
[21] The method according to [20], wherein the tumor marker is methylated DNA in the promoter region of a gene selected from the group consisting of SFRP1, SFRP2, DKK2, and hsa-mir-34b/c.
[22] The method according to any one of [14] to [21], wherein the index is a case wherein the methylated DNA in the promoter region of SFRP1 gene is more than 45%.
[23] The method according to any one of [14] to [21], wherein the index is a case wherein the methylated DNA in the promoter region of SFRP1 gene is more than 51%.
[24] The method according to any one of [14] to [21], wherein the index is a case wherein the methylated DNA in the promoter region of SFRP2 gene is more than 10%.
[25] The method according to any one of [14] to [21], wherein the index is a case wherein the methylated DNA in the promoter region of SFRP2 gene is more than 33%.
[26] The method according to any one of [14] to [21], wherein the index is a case wherein the methylated DNA in the promoter region of DKK2 gene is more than 10%.
[27] The method according to any one of [14] to [21], wherein the index is a case wherein the methylated DNA in the promoter region of DKK2 gene is more than 11%.
[28] The method according to any one of [14] to [21], wherein the index is a case wherein the methylated DNA in the promoter region of mir-34b/c gene is more than 15%.
[29] The method according to any one of [14] to [21], wherein the index is the methylated DNA in the promoter region of mir-34b/c gene is more than 18%.
[30] The method according to any one of [14] to [21], wherein the index is a case wherein the diameter of a tumor observed from the large intestinal lumen side is 20 mm or more and the methylated DNA in the promoter region of mir-34b/c gene is more than 15%.
[31] The method according to any one of [14] to [21], wherein the index is a case wherein the diameter of a tumor observed from the large intestinal lumen side is 20 mm or more and the methylated DNA in the promoter region of mir-34b/c gene is 15% or less, and the methylated DNA in the promoter region of DKK2 gene is more than 10%.
[32] The method according to any one of [14] to [21], wherein the index is a case wherein the diameter of a tumor observed from the large intestinal lumen side is 20 mm or more and the methylated DNA in the promoter region of SFRP1 gene is more than 51%, and the methylated DNA in the promoter region of SFRP2 gene is more than 10%.
[33] The method according to any one of [14] to [32], wherein the index is an index used for evaluating therapeutic effects of a drug and/or a therapeutic method.

### Effects of Invention

The specimen, kit and method of the present invention use a colonic-mucous-layer detachment fluid obtained by washing the colonic mucous layer that adheres to the mucosa of a lesion in the subjective large intestine, and are fundamentally different from conventional methods using biopsy samples of the colonic mucosa.

According to the specimen, kit, and method of the present invention that use the colonic-mucous-layer detachment fluid, different from conventional methods using biopsy samples of the colonic mucosa, a larger amount of tumor cells can be collected from the colonic mucous layer as the degree of invasion of cancer or tumor increases. Furthermore,according to the specimen, kit, and method of the present invention, as the degree of invasion of cancer or tumor increases, a larger amount of methylated DNA derived from tumor cells can be collected from the colonic mucous layer. Accordingly, the specimen, kit, and method of the present invention are useful as a specimen, kit and method for obtaining an index to diagnose the degree of invasion of tumors, which can provide various beneficial effects that had been impossible to achieve by conventional methods

Namely, since the specimen, kit, and method of the present invention use, as a material, a washing fluid that had been disposed of to date, various risks that may be caused by other test methods using an endoscope (risk of bleeding (biopsy), allergy, extended test time (magnifying endoscopy), expensive facilities (magnifying endoscopy, NBI)) do not occur, and since the collection of a specimen is easy, almost no burden is placed on physicians performing endoscopy and clinical nurses assisting them. Furthermore, in the specimen, kit, and method of the present invention, a commercially available endoscopic apparatus can be used as is, eliminating the necessity of new facility investment.

In addition, with the specimen, kit and method of the present invention, it becomes possible to evaluate a lesion as an "area" by washing its entire mucosal surface, and moreover, since the tissue is not injured as in the case of biopsy, tests can be performed for subjects whose hemostatic function and wound healing ability are decreased, or for subjects who are orally taking drugs affecting blood stanching and wound healing, such as antiplatelet drugs and anticoagulants. Since many of the subjects requiring endoscopy are relatively elderly persons, the ratio of those who taking such drugs is high; thus, such an advantage is extremely important.

According to the specimen, kit and method of the present invention, it is possible to perform cytological diagnosis and DNA diagnosis without injuring tumors prior to surgery. Accordingly, using the specimen, kit and method of the present invention, accuracy of estimating properties and degree of invasion of tumors can be significantly improved. Furthermore, using the specimen, kit and method of the present invention, it becomes possible to diagnose genetic profiles of colorectal tumors before surgery.

With the specimen, kit and method of the present invention, it is possible to detect genetic or epigenetic abnormalities of genomic DNA. Furthermore, sensitivity of various drugs including anticancer agents can be examined using specimens collected by the method of the present invention. Moreover, using the specimen and method of the present invention, evaluation of therapeutic effects of drugs and/or therapeutic methods becomes possible. With the specimen and method of the present invention, it becomes possible to obtain an index to predict recurrence.

According to the analysis of excessive methylation by a colonic-mucous-layer detachment fluid using the specimen, kit and method of the present invention, accurate diagnosis of invasive colorectal tumors by a simple and neon-invasive method becomes possible at any facilities. Thus, the specimen, kit and method of the present invention are extremely useful.

### [Brief Description of Drawings]

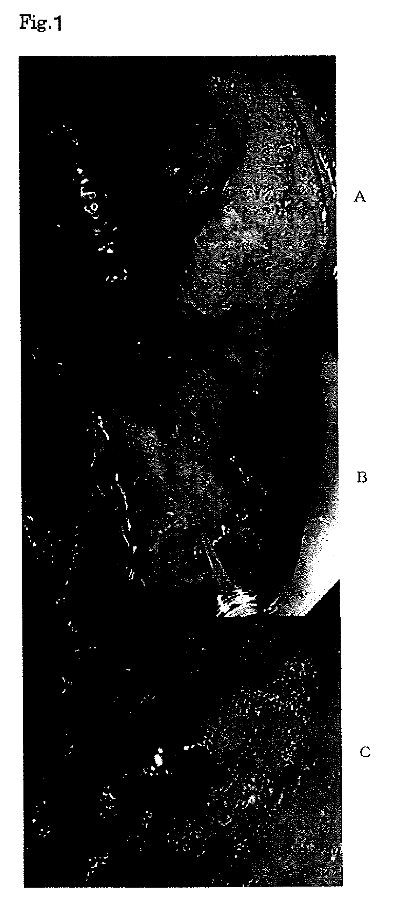

[Fig. 1] Figure 1 shows photographs showing the process of spraying a washing fluid onto the colonic mucosa and thereby detaching the mucus from the mucous layer. "A" shows a tumor part before spraying the washing fluid, "B" shows spraying the washing fluid onto the tumor part, and "C" shows the tumor part after spraying the washing fluid.

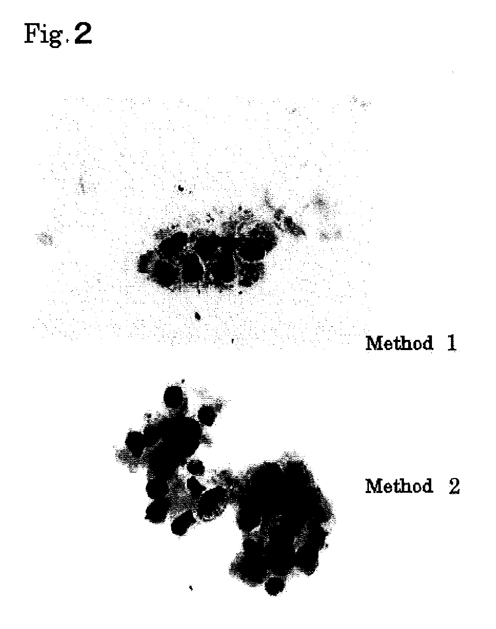

[Fig. 2] Figure 2 shows photographs of cells obtained by spraying a washing fluid onto the colonic mucous layer and thereby detaching the mucus from the mucous layer. "Method 1" shows a photograph of cells obtained by spraying water onto the colonic mucous layer and detaching the mucus from the mucous layer. "Method 2" shows a photograph of cells obtained by spraying saline onto the colonic mucous layer and detaching the mucus from the mucous layer. The cells were stained with hematoxylin-eosin.

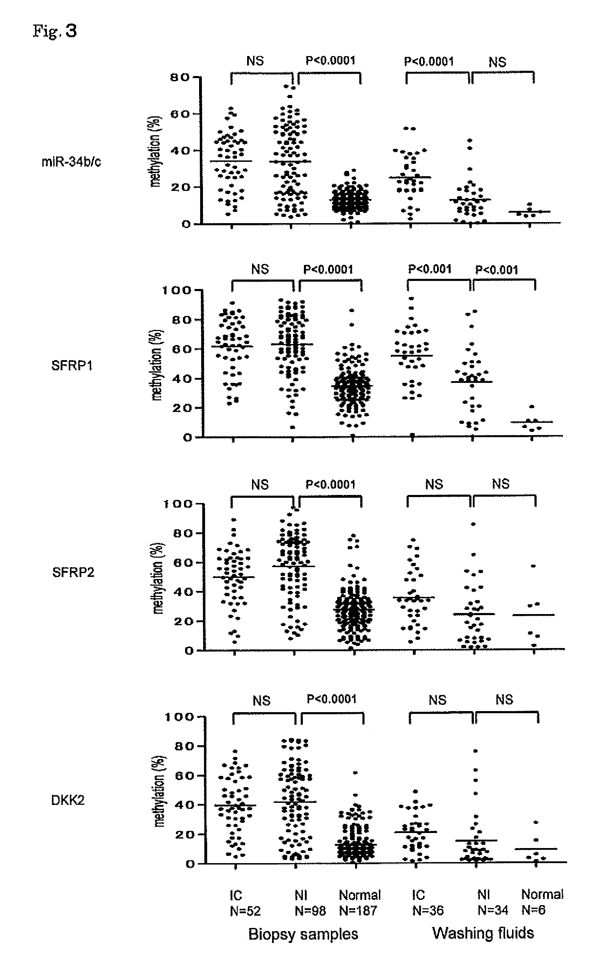

[Fig. 3] Figure 3 shows diagrams of the ratio of methylated DNA in the large intestine of patients with invasive tumor, with non-invasive tumor, and noncancer, compared between biopsy sample and colonic-mucous-layer detachment fluid. In the diagrams, each point shows each case. The vertical axis indicates the ratio of methylated DNA of CpG islands in the promoter region of each gene miR-34b/c, SFRP1, SFRP2, and DKK2. "Biopsy samples" indicates biopsy sample, "Washing fluids" indicates colonic-mucous-layer detachment fluid. "IC" indicates invasive tumor, "NI" indicates non-invasive tumor, "Normal" indicates patient of noncancer. Numerical values after "N=" indicate the number of cases. "NS" means that there is no significant difference, and numerical values after "P<" indicate the level of significance. For example, "P<0.0001" means that there is a statistically significant difference with a significance level of 0.01%. Here, horizontal lines in the diagrams show mean values.

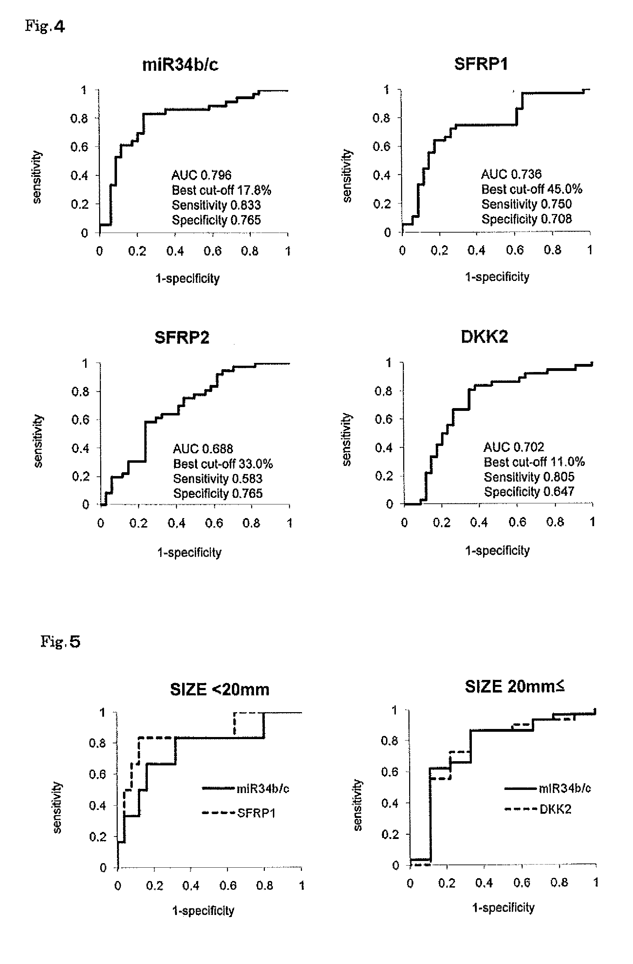

[Fig. 4] Figure 4 is a diagram showing receiver operating characteristic curves (ROC curves). By producing ROC curves and determining best cut-off values, the most appropriate threshold value for distinguishing between invasive tumors and non-invasive tumors, using the ratio of methylated DNA of each gene in a specimen (method 2) collected by spraying saline as a washing fluid, can be determined. mir-34b/c, SFRP1, SFRP2 and DKK2 indicate the gene name. The vertical axis "sensitivity" indicates "sensitivity", and the horizontal axis "1-specificity" indicates "1-specificity". "AUC" indicates the area under the ROC curve. "Best cut-off" indicates the most appropriate threshold value, i.e., "best cut-off" to distinguish between invasive tumor and non-invasive tumor. In the figure, numerical values listed to the right of "sensitivity" indicate sensitivities at the "best cut-off, and numerical values listed to the right of "specificity" indicate specificities at the "best cut-off."
[Fig. 5] Figure 5 shows ROC curves of ratio of methylated DNA when tumors were classified by their size: diameter of less than 20 mm (left diagram) and diameter of 20 mm or more (right diagram). In the left diagram, for the case of tumors with a diameter of less than 20 mm, ROC curves of the ratio of methylated DNA of each gene of mir-34b/c and SFRP1 are shown; and in the right diagram, for the case of tumors with a diameter of 20 mm or larger, ROC curves of the ratio of methylated DNA of each gene of mir-34b/c and DKK2 are shown. "Sensitivity" of the vertical axis indicates "sensitivity," and "1-specificity" of the horizontal axis indicates "1-specificity."

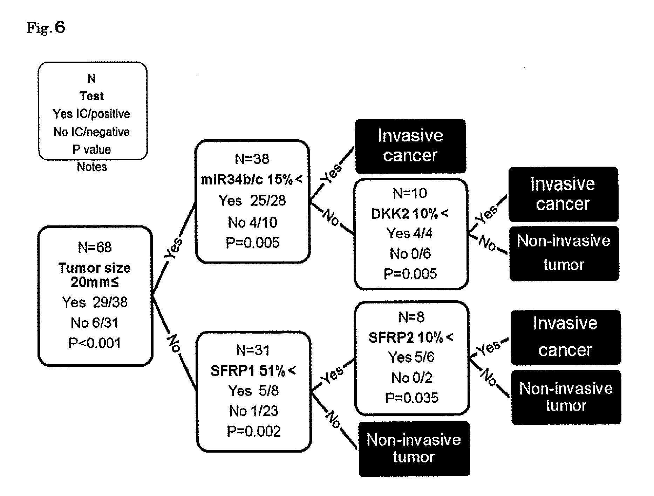

[Fig. 6] Figure 6 is a diagram showing the determinationprocess of presence/absence of invasive tumors, using the ratios of methylated DNA of multiple genes as indices. In the figure, "Tumor size 20 mm<" indicates the determination criterion that the tumor diameter is 20 mm or more. "mir34b/c 15%<" indicates the determination criterion that the ratio of methylated DNA of mir34b/c gene is more than 15%. "SFRP1 51%<" indicates the determination criterion that the ratio of methylated DNA of SFRP1 gene is more than 51%. "DKK2 10%<" indicates the determination criterion that the ratio of methylated DNA of DKK2 gene is more than 10%. "SFRP2 10%<" indicates the determination criterion that the ratio of methylated DNA of SFRP2 gene is more than 10%. "Yes" existing in the center of bar lines means that the criterion of its left side is satisfied; similarly, "No" existing in the center of bar lines means that the criterion of its left side is not satisfied. "Invasive Caner" indicates diagnostic result of invasive tumor. "Non-invasive tumor" indicates diagnostic result of non-invasive tumor. The denominator of a numerical value to the right of "Yes" in the box indicates the number of cases that satisfy the criterion in the box, and the numerator indicates the number of cases of invasive tumors among the cases satisfying the criterion in the box. The denominator of a numerical value to the right of "No" in the box indicates the number of cases that do not satisfy the criterion in the box, and the numerator of the numerical value to the right of "No" in the box indicates the number of cases of invasive tumors among the cases that do not satisfy the criterion in the box. Numerical values after "P<" indicate the level of significance. For example, "P<0.001" means that there is a statistically significant difference with a significance level of 0.1%.

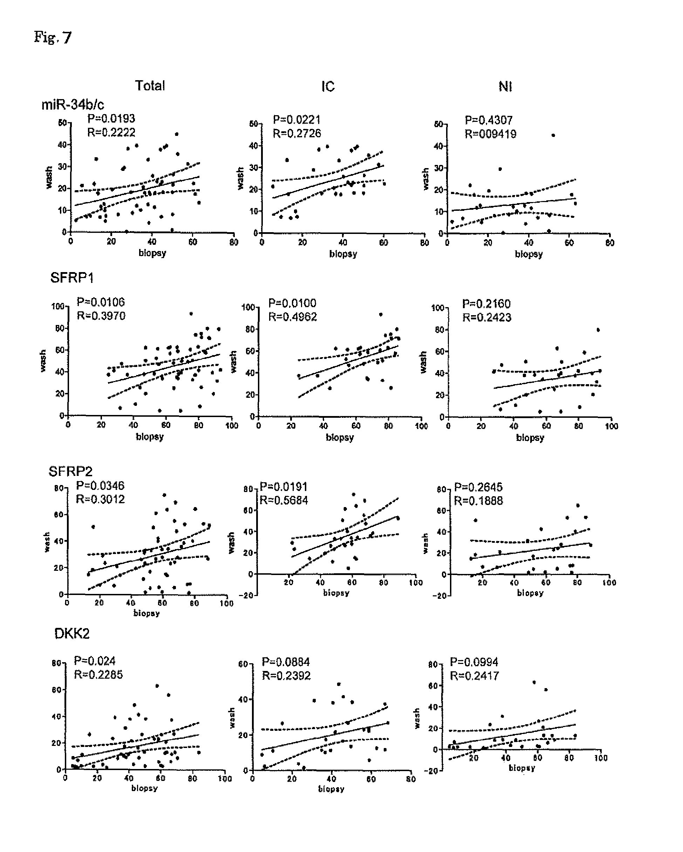

[Fig. 7] Figure 7 shows diagrams for investigating the correlation between the ratio of methylated DNA of each gene in a specimen collected by spraying saline as a washing fluid (Method 2) and the ratio of methylated DNA of each gene in biopsy sample. In the figure, each point shows each case. "wash" on the vertical axis indicates the "ratio of methylated DNA in the colonic-mucous-layer detachment fluid obtained by spraying saline onto tumors," and "biopsy" on the horizontal axis indicates the "ratio of methylated DNA in the biopsy sample." Solid lines in the figure show regression lines by analysis of covariance, and dotted lines above and below the solid lines show confidence limits with a confidence level of 95%. Numerical values to the right of "R=" indicate Pearson's correlation coefficients, and values to the right of "P=" indicate risk rates.

### [Embodiments for Carrying out the Invention]

Hereinafter, preferred embodiments of the present invention are described in detail.
The present invention relates to a specimen for detecting invasive colorectal tumors which is obtained by non-invasively detaching the colonic mucous layer, a kit for non-invasively detaching the colonic mucous layer, a method for obtaining an index to detect invasive colorectal tumors by non-invasively detaching the colonic mucous layer, and a method for obtaining an index to evaluate therapeutic effects of a drug and/or a therapeutic method by non-invasively detaching the colonic mucous layer.

The washing fluid used in the present invention is preferably a liquid that can detach the colonic mucous layer without injuring the mucosa of the large intestine, and it is more preferably an isotonic solution, and furthermore preferably saline. In addition, the washing fluid used in the present invention may comprise any additives such as pigments, antibiotics, neutral buffer compositions, chelating agents and additives for preservation, as long as the washing fluid can detach the colonic mucous layer without injuring the mucosa of the large intestine.

In the present invention, the washing fluid is sprayed onto the colonic mucous layer at a rate that can detach the colonic mucous layer without injuring the mucosa of the large intestine, and it is sprayed at a rate of preferably 2 ml/s to 10 ml/s, more preferably at a rate of 3 ml/s to 8 ml/s, and furthermore preferably at a rate of 4 ml/s to 6 ml/s.

The method for collecting the washing fluid in the present invention is not particularly limited; when an endoscopic apparatus is used, for example, a method wherein at least one specimen-collecting container that can be sealed is connected to an operating part in an aspiration tube, connector, aspiration tank or aspirator or the parts therebetween, may be included. When ease of washing and sterilization and risk of contamination are considered, the specimen-collecting container is preferably connected between the connector and the aspiration tank. In addition, from the viewpoints of operability and ease of washing, the specimen-collecting container is preferably connected in a detachable manner.

Typically, for example, the aspiration tube that connects the connector and the aspiration tank is detached from the aspiration-tank side, and this tube is connected to the inflow-side connector of the specimen-collecting container, and the outflow-side connector of said container is connected to the aspiration tank. The endoscopic apparatus is preferably equipped with a member to support the specimen-collecting container. Only one specimen-collecting container mentioned above may be connected, or multiple containers may be connected in serial or in parallel.

The tumor markers used in the present invention may be any tumor markers as long as they can detect tumor cells using a colonic mucous layer detachment fluid. For example, a histological diagnostic method of cells stained with markers such as hematoxylin-eosin, etc. may be used, preferably, tumor markers are markers of which colorectal cancer may be diagnosed by the detection, such as fetal proteins (AFP, CEA, etc.), carbohydrate antigens (CA19-9, serial Tn, etc.), ectopically-produced substances (hormones and tumor isozyme, etc.), and more preferably, oncogenes and tumor suppressor genes. Examples of oncogenes or tumor suppressor genes include cancer-related genes such as APC, K-RAS, H-RAS, N-RAS, erbB, p53, P16, BCR-ABL, CHFR, RASSF family, SFRP family, MINT Family, MGMT, RUNX family, SMAD family and PRDM family; EBV and its related genes, and CMV and its related genes, etc.; however, examples are not limited thereto; various oncogenes or tumor suppressor genes which have not yet been known at present but which will be discovered in the future may be used.
The tumor markers used in the present invention are, more preferably, the detection of methylated DNA of tumor-related genes, and most preferably, the detection of methylated DNA in the promotor region of a gene selected from the group consisting of SFRP1, SFRP2, DKK2, hsa-mir-34b/c, p16INK4A, E-cadherin, hMLH1, 14-3-3 sigma, BNIP3, CHFR, CIITA, IGFBP7, Histone H3K27, HRK, CACNA1G, COX2, DFNA5, and RASSF2.

The present invention further relates to a method for detecting tumor markers, comprising a step of extracting nucleic acid from the specimen of the present invention.

In the method of the present invention, any publicly-knownmethods may be used for extracting tumor markers from a specimen. Typically, a specimen is centrifuged, and the pellet obtained is re-suspended in an appropriate medium such as PBS and saline, digested with a protein dissolving agent such as proteinase K, deproteinized by an organic solvent such as phenol and chloroform, and nucleic acids are precipitated by ethanol, etc. to extract the nucleic acids. Concrete protocols are described in various references regarding genetic engineering (for example, Chomczynski, P., Sacchi, N.: Anal. Biochem., 162: 156-159, 1987; Masami Muramatsu and Masashi Yamamoto, Ed., New Handbook of Genetic Engineering, 4th revision, Yodosha, Oct. 2003, 20-29), and therefore they are not described in detail here.

It is preferable that the specimen is subjected to extraction treatment immediately after its collection; however, the specimen can be stored for a certain period before extraction treatment, for example approximately 12 hr, and also approximately 24 hr. Preferred storage temperature is, from the viewpoint of protection of tumor markers, preferably -80°C to 20°C, more preferably -80°C to 10°C, and particularly preferably -80°C to 4°C. When the specimen is to be stored, preferably it is stored in a re-suspended state in the above appropriate medium after centrifugation.

The extracted nucleic acid can be detected by various detection methods corresponding to the target diseasemarkers, including for example, various nucleic acid amplification methods such as PCR, nucleic acid sequence-based amplification (NASBA), transcription mediated amplification (TMA), ligase chain reaction (LCR), strand displacement amplification (SDA), loop-mediated isothermal amplification (LAMP), isothermal and chimeric primer-initiated amplification of nucleic acids (ICAN), and branched DNA, as well as southern blotting, northern blotting, RNase protection assay, microarray, dot blot, and slot blot, etc.

In particular, when the marker is epigenetic methylated DNA, it can be detected by, for example, bisulfite sequence method,methylation-specificPCR(MS-PCR),combined bisulfite restriction assay (COBRA), MS-SNuPE, bisulfite-SSCP, differential methylation hybridization (DMH), methyLight method, pyrosequencing, etc.

In addition, quantification of nucleic acids may be carried out using any known method including spectrophotometric method for measuring absorbance at maximum absorption wavelength of approximately 260 nm, and a method using various reagents that stain nucleic acids such as ethidium bromide, DAPI (4,6-diamidine-2-phenylindole), acridine orange, Mupid (registered trademark)-STAIN eye (Advance Co., Ltd.), diphenylamine reagent, Hoechst 33258 (H33258), Quant-iT PicoGreen dsDNA Reagent (Invitrogen), Quant-iT RiboGreen (registered trademark) RNA Reagent (Invitrogen), Gel Indicator RNA Staining Solution (Funakoshi), SYBR (registered trademark) Green I or II, SYBR (registered trademark) Gold, and GelRed, etc.

In the present invention, the colonic-mucous-layer detachment fluid can be obtained by spraying a washing fluid onto the colonic mucous layer without pre-washing, and preferably, can be obtained by spraying a washing fluid directly onto the site with suspected invasion of cancer or tumor.

The washing fluid used in the present invention is preferably 10-100 ml, more preferably 15-50 ml, and yet more preferably 17-30 ml, and the most preferably 20 ml. According to the present invention, a specimen for detecting invasive colorectal tumors comprising 10 µg or more DNA can be obtained, and preferably, a specimen for detecting invasive colorectal tumors comprising 12 µg or more DNA can be obtained, and furthermore preferably, a specimen for detecting invasive colorectal tumors comprising 15 µg or more DNA can be obtained.

The kit of the present invention may contain the above preferred washing fluid, and a washing-fluid spraying apparatus that can detach the colonic mucus layer without injuring the mucosa of the large intestine.

The method of the present invention for obtaining an index to detect invasive colorectal tumors may comprise a step of spraying a washing fluid onto the colonic mucous layer to detach the mucus from the mucous layer, thereby collecting a specimen comprising the detached colonic mucus in said washing fluid, a step of extracting a nucleic acid in the specimen, and a step of determining the presence or absence of a tumor marker in the nucleic acid.

In the present invention, regarding the criteria for determining the presence of invasive colorectal tumors using methylated DNA of SFRP1 gene as the index, preferably presence of an invasive colorectal tumor can be determined when the methylated DNA in the promoter region of SFRP1 gene is more than 45%, and more preferably, presence of an invasive colorectal tumor can be determined when the methylated DNA in the promoter region of SFRP1 gene is more than 51%.

In the present invention, regarding the criteria for determining the presence of invasive colorectal tumors using methylated DNA of SFRP2 gene as the index, preferably presence of an invasive colorectal tumor can be determined when the methylated DNA in the promoter region of SFRP2 gene is more than 10%, and more preferably, presence of an invasive colorectal tumor can be determined when the methylated DNA in the promoter region of SFRP2 gene is more than 33%.

In the present invention, regarding the criteria for determining the presence of invasive colorectal tumors using methylated DNA of DKK2 gene as the index, preferably presence of an invasive colorectal tumor can be determined when the methylated DNA in the promoter region of DKK2 gene is more than 10%, and more preferably, presence of an invasive colorectal tumor can be determined when the methylated DNA in the promoter region of DKK2 gene is more than 11%.

In the present invention, regarding the criteria for determining the presence of invasive colorectal tumors using methylated DNA of mir-34b/c gene as the index, preferably presence of an invasive colorectal tumor can be determined when the methylated DNA in the promoter region of mir-34b/c gene is more than 15%, and more preferably, presence of an invasive colorectal tumor can be determined when the methylated DNA in the promoter region of mir-34b/c gene is more than 18%.

According to the present invention, using the size of a tumor observed from the large intestinal lumen side and the methylated DNA of multiple genes as the indices, presence of an invasive colorectal tumor can be determined with high accuracy. For example, regarding the tumor size, when the diameter of a tumor observed from the large intestinal lumen side is 20 mm or larger, it can be determined that an invasive colorectal tumor is present. Furthermore, for example, when the diameter of a tumor observed from the large intestinal lumen side is 20 mm or larger and the methylated DNA of the promoter region of hsa-mir-34b/c gene is more than 15%, it can be determined that an invasive colorectal tumor is present. In another embodiment, when the diameter of a tumor observed from the large intestinal lumen side is 20 mm or larger and the methylated DNA of the promoter region of hsa-mir-34b/c gene is 15% or less and the methylated DNA of the promoter region of DKK2 gene is more than 10%, it can be determined that an invasive colorectal tumor is present. Moreover, in another embodiment, when the diameter of a tumor observed from the large intestinal lumen side is 20 mm or larger and the methylated DNA of the promoter region of SFPR1 gene is more than 51% and the methylated DNA of the promoter region of SEPA2 gene is more than 10%, it can be determined that an invasive colorectal tumor is present.

Furthermore, the method for obtaining an index to detect invasive colorectal tumors of the present invention may be used as a method for evaluating therapeutic effects of a drug and/or a therapeutic method.

For example, by comparing the marker level prior to treatment with the marker level after treatment, or by comparing the marker level at a certain time point during the treatment with the marker level at the certain time point after the previous time point during the treatment, and when the progression of the target disease is slowed or stopped, or the target disease is eliminated, then it can be evaluated that therapeutic effects are observed; when the progression of the target disease is not affected, then it can be evaluated that no therapeutic effects are observed.

The method of the invention can also be used for monitoring the presence/absence of recurrence of the disease after completion of treatment. In this case, a specimen is collected from a subject at regular intervals after completion of treatment, and presence/absence of the disease is checked by the same method as described above based on marker levels.

Unless otherwise stated in this specification, scientific and technical terms used in relation to the present invention shall have the meaning that is usually understood in the art. In general, as used herein, terms and technologies used with respect to cells and tissue cultures, molecular biology, immunology, microbiology, gene and protein and nucleic acid chemistry shall be those well-known and commonly used in the art. In addition, unless otherwise stated, the methods and technologies of the present invention are carried out in accordance with well-known conventional methods in the art as described in various general and specialized references cited and discussed herein. Examples of such references include Sambrook et al., Molecular Cloning: A Laboratory Manual, 2nd ed., Cold Spring Harbor Laboratory Press (1989) and Sambrook et al., Molecular Cloning: A Laboratory Manual, 3rd ed., Cold Spring Harbor Press(2001); Ausubel et al., Current Protocols in Molecular Biology, Greene Publishing Associates (supplements in 1992 and 2000) ; Ausubel et al., Short Protocols in Molecular Biology: A Compendium of Methods from Current Protocols in Molecular Biology-4th Ed., Wiley & Sons (1999); Harlow and Lane, Antibodies: A Laboratory Manual, Cold Spring Harbor Laboratory Press (1990); and Harlow and Lane, Using Antibodies:A Laboratory Manual,ColdSpring Harbor Laboratory Press (1999).

Terms used with respect to analytical chemistry, synthetic organic chemistry and medicinal chemistry as described herein, and the experimental procedures and techniques thereof are those that are well known and normally used in the art. Standard techniques are used in chemical synthesis, chemical analysis, preparation, formulation and delivery of drugs, and treatment of subjects.

Incidentally, the term "case" in the present invention refers to a subject that is a human patient.

As used herein, unless otherwise stated, the name of a protein is expressed by an alphabetical (or alphanumeric) name of the gene followed by "protein, " and the gene encoding the protein is expressed by the above alphabetical (or alphanumeric) name of the gene alone, or by the alphabetical (or alphanumeric) name of the gene followed by "gene." The above "gene" means any one of the genomic gene, messenger RNA or cDNA; when any one of them is to be specified, it should be specifically expressed.

In the present invention, "subject" means any individual organism having the large intestine, and is preferably an animal, more preferably a mammal, for example humans, nonhuman primates, companion animals such as dogs and cats, industrial animals such as cows, horses, goats, sheep, pigs, and particularly preferably a human. In the present invention, the subject may be a healthy subject, or may be suffering from a certain disease, or may be under treatment or after treatment of disease.

In the present invention, "tumor" refers to a condition of abnormal cell growth, and among tumors, malignant tumors indicate "cancer."

In the present invention, "invasion depth" refers to a degree of invasion of cancer (colorectal cancer) from the lumen side to deep layers, which is synonymous with "degree of invasion."

In the present invention, "invasive colorectal tumor detection," "detection of invasive colorectal tumor," and "method for detecting invasive colorectal tumor" mean a method for obtaining an index used to detect whether or not an invasive colorectal tumor is present.

As used herein, "epigenetic" shall have a usual meaning in the art of the present invention, and is exemplified by, for example, that gene expression is regulated by an acquired modification to chromatin without changes in the DNA sequence. Examples of acquired modification to chromatin include, in addition to methylation of DNA bases, chemical modification such as methylation, acetylation, and phosphorylation of histone.

In the present invention, "amount of a tumor marker" refers to: histological evaluation scores, concentrations of fetal proteins, carbohydrate antigens, or ectopically-produced substances in the body fluid such as blood, etc., amounts of mutated oncogenes or tumor suppressor genes contained in a specimen, and amounts of methylated DNA of tumor-related genes contained in a specimen.

In the present invention, "DNA methylation" refers to that the cytosine in CpG islands in the promoter region of any genomic gene is methylated.

In the present invention, "excessive DNA methylation" or "excessive methylation" refers to the condition wherein CpG islands in the promoter region of any genomic gene are excessively methylated compared to normal cells.

In the present invention, "methylation of CpG island" refers to the DNA methylation in CpG islands existing in the promoter region of any genomic gene.

In the present invention, "methylated DNA" refers to methylated cytosine in CpG islands of the promoter region of any genomic gene, and the "ratio of methylated DNA" refers to the ratio of "methylated DNA" among the cytosine of all CpG islands in said region.

In the present invention, the "amount of methylated DNA" refers to the amount of methylated cytosine in CpG islands of the promoter region of any genomic gene. The "amount of methylated DNA" may be expressed as the "ratio of methylated DNA."

In the present invention, "invasive tumor" refers to cancer that has invaded through the muscular layer of the mucosa into the submucosa or deeper layers.

In the present invention, "non-invasive tumor" refers to cancer that is confined to the mucosa.

In the present invention, "noncancer" is used to refer to a subject who is not affected with colorectal cancer, or refer to a specimen collected from a subject who is not affected with colorectal cancer.

In the present invention, "specimen for detecting invasive colorectal tumors" refers to a specimen for obtaining an index to detect presence or absence of invasive colorectal tumors, wherein the specimen is obtained by spraying a washing fluid onto the colonic mucous layer and detaching the mucus from the mucous layer, and wherein the specimen comprises the detached mucus in said washing fluid.

In the present invention, "colonic mucous layer" refers to the mucous layer that exists on the surface of the mucosa in the large intestinal lumen.

In the present invention, "colonic-mucous-layer detachment fluid" refers to a fluid that is obtained by spraying a washing fluid onto the colonic mucous layer and detaching the mucus from the mucous layer.

In the present invention, "physiological isotonic solution" refers to an isotonic solution with nearly neutral pH, which does not comprise a component that irritates the colonic mucosa.

In the present invention, both "hsa-mir-34b/c" and "mir-34b/c" mean a genomic gene common to micro RNA 34b and micro RNA 34c. When "mir-34b/c" is referred as a general term, it may indicate transcribed micro RNA; therefore, the term "hsa-mir-34b/c" is used to emphasize that this refers to a genomic gene.

In the present invention, in the representation of a primer sequence used for detecting methylated DNA, the base represented by "Y" means thymine (T) whenunmethylatedcytosine in CpG islands is to be detected, and it means cytosine (C) when methylated cytosine in CpG islands is to be detected.
In the present invention, in the representation of a primer sequence used for detecting methylated DNA, the base represented by "R" means adenine (A) or guanine (G), so that primers wherein "R" is adenine (A) and primers wherein "R" is guanine (G) are used in a mixture.

In the present invention, "ROC curve" refers to a receiver operating characteristic curve.

The ROC curve can be used for determining a threshold value to judge positive result or negative result, when a specimen is to be tested positive/negative for a specific disease, based on test results expressed by continuous numerical values.

In the ROC curve, "sensitivity" indicates the ratio of specimens that were correctly tested positive which must be tested positive, and "specificity" indicates the ratio of specimens that were correctly tested negative which must be tested negative. In general, when a specimen that must be tested positive was correctly tested positive, this is defined as "true positive; " when a specimen that must be tested positive was erroneously tested negative, this is defined as "false-negative; " when a specimen that must be tested negative was correctly tested negative, this is defined as "true negative;" when a specimen that must be tested negative was erroneously tested positive, this is defined as "false-positive." The "sensitivity" can be calculated by dividing the number of true positive by the summation of the number of true positive and the number of false-negative. In addition, the "specificity" can be calculated by dividing the number of true negative by the summation of the number of false-positive and the number of true negative.

"AUC" indicates the area under a ROC curve. "auk" is a value between 0.5 and 1.0, and as the value approaches 1.0, this indicates that the test method is superior.

"Best cut-off," that is the most appropriate threshold value to differentiate invasive tumor from non-invasive tumor, can be obtained as the point on the ROC curve located at the shortest distance from the upper-left point on the ROC curve at which "sensitivity" is 1 and "1-specificity" is 0.

[Table 1]

**Table 1. Characteristics of subjects from which specimens were collected.**

| Tumor | Hyperplastic polyp | Adenoma | Early cancer | Advanced cancer |
|---|---|---|---|---|
| Age (mean±SD) | 63.2 | 66.047 | 68.21 | 67.27 |
| Male | 5 | 52 | 15 | 32 |
| Female | 5 | 11 | 4 | 16 |
| | | | | |
| Endoscopic findings | | | | |
| Protruded type | 0 | 32 | 7 | |
| Flat type | 10 | 29 | 7 | |
| Depressed type | 0 | 1 | 3 | |
| Bormann type 1 | | | | 7 |
| Bormann type 2 | | | | 31 |
| Bormann type 3 | | | | 6 |
| Bormann type 4 | | | | 0 |
| Others | | | | 4 |
| | | | | |
| Histologic grade | | | | |
| Mild-moderate dysplasia | | 51 | | |
| Severe dysplasia | | 5 | | |
| | | | | |
| Differentiation | | | | |
| Well tub1 tub2 | | | 19 | 45 |
| Poor sig | | | 0 | 1 |
| | | | | |
| Tumor size | | | | |
| <20 mm | 10 | 57 | 2 | 0 |
| 20 mm ≤ | 0 | 6 | 17 | 46 |
| | | | | |
| Clinical stage | | | | |
| Stage 0 | | | 12 | 0 |
| Stage 1 | | | 5 | 8 |
| Stage 2 | | | 2 | 32 |
| Stage 3 | | | 0 | 5 |
| Stage 4 | | | 0 | 1 |

[Table 2]

**Table 2. Comparison of cases and DNA contents between specimens obtained by Method 1 and specimens obtained by Method 2.**

| No. of cases | 11 | | | | 37 | | | |
|---|---|---|---|---|---|---|---|---|
| | Biopsy | Colonic-mucous-layer | | | Biopsy | Colonic-mucous-layer | | |
| | | detachment fluid (Method 1) | | | | detachment fluid (Method 2) | | |
| | | Total | Invasive tumor | Non-invasive tumor | | Total | Invasive tumor | Non-Invasive tumor |
| Amount of DNA | 21.28± 15.52 | 15± 11.12 | | | 17.08± 13.0 | 16.53± 16.88 | | |
| No. of diagnos ed cases | | 2 | 1 | 1 | | 17 | 11 | 6 |
| No. of not diagnos ed cases | | 9 | 7 | 2 | | 20 | 7 | 13 |
| Ratio of diagnos ed cases | | 18.2% | 12.5% | 33.3% | | 45.9% | 31.6% | 61.1% |

[Table 3]

**Table 3. Comparison between cases of biopsy sample and cases of colonic-mucous-layer detachment fluid collected by Method 2.**

| | | Biopsy | | | | Colonic-mucous-layer detachment fluid (Method 2) | | | |
|---|---|---|---|---|---|---|---|---|---|
| | | Invasive tumor | Non-Invasive tumor | Sig. dif. | Normal tissue | Invas ive tumor | Non-Invas ive tumor | Sig. dif. | Normal tissue |
| No. of cases | | 52 | 98 | | 187 | 36 | 34 | | 6 |
| Ave. age | | 67.4 | 66.7 | None | 67.2 | 68 | 63.97 | None | 61.67 |
| Sex | Male | 16 | 24 | None | 126 | 22 | 25 | None | 3 |
| | Female | 36 | 74 | None | 61 | 14 | 9 | None | 3 |
| Tumor size | <20mm | 9 | 77 | | | 7 | 17 | | |
| | 20mm< | 43 | 21 | | | 29 | 8 | | |
| Histo-logical findings | Hyperplastic or inflammatory | | 15 | | | | 3 | | |
| | Tubular adenoma | | 29 | | | | 10 | | |
| | Ductal chorioa denoma | | 28 | | | | 7 | | |
| | High grad dysplas ia | | 26 | | | | 14 | | |
| | Cancer | 52 | | | | 36 | | | |

[Table 4]

**Table 4. Detection rate of K-RAS mutation.**

| | Invasive tumor | Non-invasive tumor | All specimens |
|---|---|---|---|
| Biopsy sample | 9 | 6 | 14 |
| Mucous detachment fluid (Method 2) | 7 | 2 | 8 |
| Concordance ratio | 77.7% | 33% | 62% |

### Examples

Hereafter, the present invention is described specifically; however, the invention is not limited to the following examples.

### <Example 1> Investigation of collection method of specimens

The specimens were collected during November 2008 and June 2009 in the Digestive Organs and Endoscopy Center of the Akita Red Cross Hospital. The subjects from which the specimens were collected include patients who have had colonic adenomas for a long time and who took colorectal endoscopy for follow-up purposes, or patients who took the same for the purpose of pre-operation of colorectal cancer surgery, or those who took the same for detailed examination by reason of positive results of fecal occult blood tests in check-ups. Biopsy was carried out for 52 specimens of invasive tumor, 98 specimens of non-invasive tumor, and 187 specimens of normal mucosa. Mucous-layer detachment fluid was collected for 34 specimens of invasive tumor, 36 specimens of non-invasive tumor, and 6 specimens of normal mucosa. Table 1 shows characteristics of subjects from which specimens were collected. "Hyperplastic polyp," "adenoma," "early cancer," and "advanced cancer" were determined by histopathological diagnosis. Classification of protruded type, flat type, depressed type, Bormann type 1, Bormann type 2, Bormann type 3 and Bormann type 4 was in accordance with the "General Rules of Colorectal Caner". Degree of dysplasia and degree of differentiation were determined in accordance with histopathological diagnosis. Tumor size was determined by measuring pathological specimens. Determination of clinical stage was in accordance with the General Rule and TNM classification.

In order to investigate collection method of specimens, specimens were collected by the following two methods: (Method 1) for 13 cases of colorectal cancer, tap water was sprayed onto the tumor site at a rate of 5 m1/s by a colon fiberscope using directly a 50-ml syringe barrel, and 10 ml of the water comprising detached mucous layer was collected; (Method 2) for 46 cases of colorectal cancer, 20 ml of saline was sprayed onto the tumor site at a rate of 5 ml/s by a Pyoktanin-spraying tube (NT tube, Olympus), and 10 ml of the water comprising detached mucous layer was collected. The number of subjects from which specimens were collected by Method 1 was 11 cases, and the number of subjects from which specimens were collected by Method 2 was 37 cases. Figure 1 shows a photograph of the lumen of the large intestine before spraying saline onto the tumor site using a Pyoktanin-spraying tube (Fig. 1A), a photograph of the lumen of the large intestine during spraying (Fig. 1B), and a photograph of the lumen of the large intestine during spraying (Fig. 1C).

The obtained specimens were centrifuged at 1500 rpm for 10 min, cells were precipitated, supernatant was discarded, and a part of the cells were formalin-fixated and stained with hematoxylin-eosin, then morphology of the cells was observed. Cytological diagnosis of colonic-mucous-layer detachment fluid of the large intestine (21 cases of advanced cancer, 5 cases of early cancer, 15 cases of adenoma) was carried out and pathological diagnosis of colorectal tumors was compared with the cytological diagnosis of colonic-mucous-layer detachment fluid. The results of the biopsy agreed with those of cytological diagnosis in 10 cases of advanced cancer (concordance rate: 10%), 0 cases of early cancer (concordance rate: 0%), 4 cases of adenoma (concordance rate: 26.6%). These results suggest that colorectal cancer cells are floating in the colonic-mucous-layer detachment fluid, and in particular, mucosa detachment fluids from advanced colorectal cancer markedly comprise tumor cells. Figure 2 shows photographs of cells in the specimens collected by either Method 1 or Method 2. In Method 1, the number of cells is small and denaturation of cells is strong. In Method 2, denaturation becomes weak and a case was observed which could be diagnosed as adenocarcinoma by cytological diagnosis. In addition, cytological diagnosis of some of the cases exhibited an image wherein the number of cells was small and the degree of denaturation of cells was large by Method 1, and an image wherein the number of cells was large and the degree of denaturation of cells was small by Method 2. Thus, it is considered that by Method 2, tumor cells of the mucous layer can be efficiently collected and abnormal methylation of tumor cells can be analyzed.

Furthermore, DNA was extracted from the above centrifuged specimens, and the amounts of DNA obtained were compared. As shown in Table 2, no large difference was observed in the DNA content between the specimens obtained by Method 1 and the specimens obtained by Method 2. The amounts of DNA in the specimens collected by Method 1 are 15.00±11.12 µg for colonic mucous layer detachment fluids, and 21.28±15.52 µg for biopsy samples. In contrast, the amounts of DNA in the specimens collected by Method 1 are 16.53+16.88 µg for colonic mucous layer detachment fluids, and 17.08+13.00 µg for biopsy samples. No difference was observed in the amount of DNA obtained between Method 1 and Method 2, and no difference was observed in the amount of DNA obtained between the colonic-mucous-layer detachment fluids and the tissues.

Meanwhile, DNA extraction was performed as follows. After centrifugation of a specimen, supernatant was removed and the pellet was re-suspended in 4.5 ml of SEDTA. To this suspension, 0.5 ml of 10% SDS and 50 ml of 20 mg/ml proteinase K (TAKARA BIO INC., Code No. 9033) were added, and incubated at 55°C for 1 hr. 5 ml of phenol (UltraPure Buffer-Saturated Phenol, Invitrogen Life Technologies) were added, and after mixing by rolling-over, incubated at 4°C for 12 hr. Then, the specimen was centrifuged at 4°C for 15 min and supernatant was discarded, and the pellet was suspended in 10 ml of 70% ethanol, centrifuged at 2700 rpm and 4°C for 15 min, supernatant was discarded, and the resulting substance was dissolved in 200 ml of purified water, giving a sample for DNA analysis.

With respect to the amount of DNA, the amount of collected DNA contained in a specimen was measured by spectrophotometric method. Spectrophotometric method was performed using a NanoDrop ND-1000 spectrophotometer (AGC Techno Glass Co., Ltd.) in accordance with the manufacturer's manual.

For the same subject, histopathological examination was performed, and diagnosis of invasive tumor is made for cases wherein the invasion is observed in the submucosa or below, and diagnosis of non-invasive tumor is made for cases wherein noncancer is determined or cancer is limited to the mucosa. In addition, using the specimens obtained by Method 1, invasive tumor or non-invasive tumor was determined by histopathological analysis. Table 2 summarizes these results. With the specimens collected by Method 1, only 12.5% of invasive tumors and 33.3% of non-invasive tumors were diagnosed correctly, whereas with the specimens collected by Method 2, 31.6% of invasive tumors and 61.1% of non-invasive tumors were diagnosed correctly. Thus, it has been demonstrated that the specimens collected by Method 2 were superior as specimens for detecting invasive colorectal cancer to the specimens collected by Method 1.

Furthermore, in order to compare biopsy samples with colonic-mucous-layer detachment fluid collected by Method 2, characteristics of the cases from which specimens were obtained were shown in Table 3. No differences in the average age and numbers of males and females were observed between the cases from which biopsy samples were obtained and the cases from which colonic-mucous-layer detachment fluids were obtained by Method 2. In Table 3, determination of invasive tumor and non-invasive tumor was carried out by histopathlogical examination. From these results, no significant differences due to gender and age were observed. The average amounts of DNA obtained from the colonic-mucous-layer detachment fluids by Method 2 and f rom biopsy of colorectal tumors were 12.28±1.24 mg (N=87) and 15.86±0.633 mg (N=365), respectively, demonstrating that colonic-mucous-layer detachment fluid obtained by colonoscopy comprises a sufficient amount of DNA for analysis.

### <Example 2> Measurement of methylated DNA and measurement of K-RAS mutation

Next, methylated DNA was measured for all cases.
First, colonoscopy was performed and when a tumor was observed by colonoscopy of the entire large bowel, a washing fluid was sprayed by a Pyoktanin-spraying tube (NT tube, Olympus) from an adjacent position of the tumor to detach the mucus from the surface of the lumen of the large intestine. At this time, the washing fluid was sprayed onto the colonic mucous layer at a rate of 5 ml/s.

Then, after observation by a magnified endoscope (CF-260AZI), background normal mucosae at a cancerous site of the tumor, adenoma site, and periphery of the tumor (within 1 cm) were collected by biopsy, and treated with EMR or marking, etc. The collected specimens were stored in endfresh, and after frozen storage, DNA extraction by phenol-chloroform method was performed.

The biopsy samples and colonic-mucous-layer detachment fluids collected were subjected to DNAextraction, then treated with sodium bisulfite using Epitect bisulfite kit from QIAGEN, in accordance with the protocol described in its handling manual "EpiTect (registered trademark) Bisulfite Protocol and Trouble Shooting" and "EpiTect (registered trademark) Bisulfite Handbook," and PCR was carried out using 1 µl of this bisulfite DNA.
Regarding all the primers, primers for detecting methylated DNA of SFRP1, SFRP2, DKK2 and micro RNA 34b/c, for whichmethylation in colorectal cancer was reported, were used.

In concrete terms, for mir-34b/c gene, DNA with SEQ ID NO 1 was used as the forward primer, DNA with SEQ ID NO 2 was used as the reverse primer, and DNA with SEQ ID NO 3 was used as the pyrosequencing primer.

In addition, for SFRP1 gene, DNA with SEQ ID NO 4 was used as the forward primer, DNA with SEQ ID NO 5 was used as the reverse primer, and DNA with SEQ ID NO 6 or 7 was used as the pyrosequencing primer.

In addition, for SFRP2 gene, DNA with SEQ ID NO 8 was used as the forward primer, DNA with SEQ ID NO 9 was used as the reverse primer, and DNA with SEQ ID NO 10 was used as the pyrosequencing primer.

Similarly, for DKK2 gene, DNA with SEQ ID NO 11 was used as the forward primer, DNA with SEQ ID NO 12 was used as the reverse primer, and DNA with SEQ ID NO 13 or 14 was used as the pyrosequencing primer.

In all the PCR assays, 50 cycles of the following steps were performed: a denaturation step at 95°C for 30 s, then an annealing step at 60°C for 30 s, and an extension step at 72°C for 30 s were carried out. Thereafter, DNA methylation levels were quantitatively measured by a pyrosequencer, and an averaged value of respective methylation levels at the CpG site upstream of the primer was adopted.

With respect to mutation of K-RAS gene, codon 12 and codon 13 were measured using a pyrosequence method.
Mutation of K-RAS gene was measured using a KRAS detection kit PyroMark KRAS v2.0 (4 x 24) (Catalogue No. 970452) from QIAGEN, in accordance with the protocol described in its handling manual "PyroMark (registered trademark) KRAS v2.0 Handbook."

In the biopsy samples, methylation levels of SFRP1, SFRP2, DKK2 and mir34b/c show tendencies of gradual increase in non-cancer parts, adenoma and cancer parts, but no significant difference was observed. In contrast, in the analysis of methylated DNA using mucosal detachment fluid, in advanced cancers and in non-cancerous parts, methylated DNA of SFRP1 is 52.5% in cancer parts and 11.0% in non-cancerous parts, showing a significant difference with a significance level of P<0.001. The level of methylated DNA of microRNA34b/c is 26.39% in cancer part and 5.33% in non-cancerous part, showing a significant difference with a significance level of P<O. 0007. Similarly, when early cancer is compared with progressive cancer, the level of methylated DNA of SFRP1 is 41.2% in early cancer and 11.0% in non-cancerous part, showing a significant difference with a significance level of p=0.0043. The level of methylated DNA of microRNA34b/c is 23.38% in early cancer and 5.33% in non-cancerous part, showing a significant difference with a significance level of p=0.0220. When adenoma is compared with non-cancer part, the level of methylated DNA of SFRP1 is 32.6% in adenoma and 11.0% in non-cancer part, showing a significant difference with a significance level of p=0.0089. The level of methylated DNA of microRNA34b/c is 17.17% in adenoma and 5.33% in non-cancerous part, showing a significant difference with a significance level of p=0.0306. In addition, regarding the relationship between invasion depth of cancer and methylation, a significant difference was observed in cases of invasion depth of m or mp or deeper as follows: the level of methylated DNA of SFRP1 was 33.39% for the invasion depth of m, and 57.17% for the invasion depth of mp, showing a significant difference with a significance level of P=0.0230. The level of methylated DNA of micrRNA34b/c was 15.62% for the invasion depth of m, and 29.36% for the invasion depth of mp, showing a significant difference with a significance level of P=0.0477. A correlation between the whole biopsy and the colonic-mucous-layer detachment fluid was observed only for the level of methylated DNA of microRNA34b/c, with a significance level of P<0.001 and a correlation coefficient R of 0.4296±0.1000.

Presence/absence of K-RAS gene mutation was investigated using a part of specimens derived from the same lesion obtained as in Table 3, and the results are shown Table 4. The results show that invasive tumors can be detected in 77.7% of the specimens by both biopsy samples and mucous detachment fluid collected by Method 2. Here, the total numbers of specimens do not agree, because in some cases, while K-RAS gene mutation was detected in biopsy, it was not detected in mucous detachment fluid. Such a case was particularly found in a large number for non-invasive tumors.

### <Example 3> Endoscopic classification

Tumor morphology of hyperplastic polyp, adenoma, and early cancer was classified into protruded type (0-I), flat type (IIa, LST),or depressed type (IIc, IIa+IIc) by colorectal endoscopy. Early cancer was defined as those wherein cancer invasion reaches the submucosa, regardless of venous invasion or lymphatic invasion. Borrmann classification was applied to advanced cancer. All the colorectal tumor surgery and EMR specimens underwent clinical diagnosis in accordance with WHO classification, at the Pathological Department of the Akita Red Cross Hospital.

### <Example 4> Cytological diagnosis

Regarding cytological diagnosis of colonic-mucous-layer detachment fluid, the colonic-mucous-layer detachment fluids collected at the Digestive Organ Center of the Akita Red Cross Hospital were stored in ThinPrep (registered trademark) PreservCyt Solution Vials (20 ml, prefilled/Box of 50 vials, order No. 0234005, CYTYC Corporation), formalin-fixated after centrifugation, and hematoxylin-eosin stained; then cytological diagnosis was conducted.

### <Example 5> Statistical analysis

All statistical analysis and graph making were carried out by PRISM version 5 for Windows (Japanese version). With respect to methylation levels, an average of methylation levels at each CpG site was determined to be the methylation level of that specimen, and examined in terms of clinical diagnosis and invasion depth. Regarding statistical significance, one-way analysis of variance was carried out in each group. As for correlation, t-test was conducted.

### <Example 6> ROC curve

ROC curves were produced to determine threshold values of methylated DNA of each gene of mir34b/c, SFRP1, SFRP2 and EKK2, used as an index to detect invasive colorectal cancer (Fig. 4). Best cut-off values of mir34b/c, SFRP1, SFRP2 and EKK2 were 17.8%, 45%, 33% and 11%, respectively.

### <Example 7> ROC curve for the cases wherein tumor diameter is less than 20 mm or 20 mm or more

ROC curves were produced for the case where the tumor diameter is less than 20 mm, and for the case where tumor diameter is 20 mm or more (Fig. 5). When the tumor diameter measured from the large intestinal lumen side is less than 20 mm, an ROV curve using methylated DNA of mir-34b/c gene and SFRP1 gene as indices was produced (Fig. 5, left figure). Tn addition, when the tumor diameter measured from the large intestinal lumen side is 20 mm or more, an ROV curve using methylated DNA of mir-34b/c gene and DKK2 gene as indices was produced (Fig. 5, right figure). These results show that differentiation of invasive tumor from non-invasive tumor when the tumor diameter is 20 mm or less is preferably carried out using methylated DNA of mix34b/c gene or SFRP gene as the index, and that differentiation of invasive tumor from non-invasive tumor when the tumor diameter is 20 mm or more is preferably carried out using methylated DNA of mir34b/c gene or DKK2 gene as the index.

### <Example 8> Diagnostic flow chart wherein tumor size and methylated DNA are combined

A flow chart for diagnosing presence/absence of invasive tumors with the highest accuracy, by combining measurement of tumor diameter from the large intestinal lumen side and measurement of methylated DNA, was made (Fig. 6). Using this flow chart, diagnosis of invasive tumor and non-invasive tumor can be made by starting from the leftmost box, wherein the judgment of tumor diameter of 20 mm or more (Yes) or that of less than 20 mm (No) is made, then by proceeding to the next box in accordance with the judgment criteria.

### <Example 9> Correlation of methylated DNA in colonic-mucous-layer detachment fluid and methylated DNA in biopsy sample

Regarding both invasive tumor and non-invasive tumor, whether the ratio of methylated DNA of each gene mir34b/c, SFRP1,SFRP2and DKK2 in colonic-mucous-layer detachment fluid correlates with that in biopsy sample was investigated. As a result, in invasive tumors, the ratio of methylated DNA of each gene of mir34b/c, SFRP1, and SFRP2 in colonic-mucous-layer detachment fluid significantly correlated with that in biopsy sample with a risk rate of 3% or less. Furthermore, in entire tumors including invasive tumors and non-invasive tumors, the ratio of methylated DNA of each gene mir34b/c, SFRP1, SFRP2 and DKK2 in colonic-mucous-layer detachment fluid significantly correlated with that in biopsy sample with a risk rate of 5% or less.

### Industrial applicability

The specimen, kit and method of the invention is a revolutionary invention that can provide a method for obtaining an index to non-invasively diagnose invasiveness or invasion depth of colorectal cancer or colorectal tumor by spraying a washing fluid onto the subject's colonic mucous layer to detach the mucus from the mucous layer, and collecting the detached mucous together with the washing fluid. By utilizing the specimen, kit and method of the invention, invasiveness or invasion depth of colorectal cancer or colorectal tumor can be diagnosed non-invasively with high accuracy; thus they are useful for selecting a therapeutic method of colorectal cancer. Furthermore, by utilizing the specimen, kit and method of the invention, sensitivity to various drugs including anti-cancer agents can be investigated in advance. By the specimen, kit and method of the invention, an index for predicting the recurrence can be obtained. Accordingly, the specimen, kit and method of the invention are industrially applicable.

## Claims

1. A specimen for detecting invasive colorectal cancer, comprising a washing fluid that is obtained by spraying the washing fluid onto the colonic mucous layer to detach the colonic mucus, and collecting the detached colonic mucus together with the washing fluid.

2. The specimen according to Claim 1, wherein the colonic mucous layer is a colonic mucous layer that comprises a tumor site.

3. The specimen according to Claim 1 or 2, wherein the washing fluid is a physiological isotonic solution.

4. The specimen according to Claim 3, wherein the physiological isotonic solution is saline.

5. The specimen according to any one of Claims 1 to 4, wherein the spraying is carried out at a rate of 2-10 ml/s.

6. The specimen according to any one of Claims 1 to 5, wherein the detection is to detect a tumor marker.

7. The specimen according to any one of Claims 1 to 6, wherein the detection of a tumor marker is to detect methylated DNA.

8. The specimen according to any one of Claims 1 to 7, wherein the detection of methylated DNA is to detect methylated DNA in the promoter region of a gene selected from the group consisting of SFRP1, SFRP2, DKK2, hsa-mir-34b/c, p16INK4A, E-cadherin, hMLH1, 14-3-3 sigma, BNIP3, CHFR, CIITA, IGFBP7, Histone H3K27, HRK, CACNA1G, COX2, DFNA5, and RASSF2.

9. The specimen according to Claim 8, wherein the detection of methylated DNA is to detect methylated DNA in the promoter region of a gene selected from the group consisting of SFRP1, SFRP2, DKK2, and hsa-mir-34b/c.

10. The specimen according to any one of Claims 1 to 9, wherein the washing fluid is sprayed without pre-washing.

11. The specimen according to Claim 2, wherein the tumor site is a tumor site suspected of tumor invasion.

12. The specimen according to any one of Claims 1 to 11, wherein the specimen comprises 10 µg or more of DNA.

13. A kit for detecting invasive colorectal cancer, containing a washing-fluid spraying apparatus for spraying a washing fluid onto the colonic mucous layer to detach the colonic mucus, and a washing fluid.

14. A method for obtaining an index to detect invasive colorectal tumor, comprising:
a step of spraying a washing fluid onto the colonic mucous layer to detach the colonic mucus,
a step of collecting the detached colonic mucus together with the washing fluid,
a step of determining the amount of a tumor marker in the collected washing fluid, and
a step of obtaining an index to determine the presence of invasive colorectal tumor based on the amount of the tumor marker.

15. The method according to Claim 14, wherein the colonic mucous layer is a colonic mucous layer that comprises a tumor site.

16. The method according to Claim 14 or 15, wherein the washing fluid is a physiological isotonic solution.

17. The method according to Claim 16, wherein the physiological isotonic solution is saline.

18. The method according to any one of Claims 14 to 17, wherein the spraying is carried out at a rate of 2-10 ml/s.

19. The method according to any one of Claims 14 to 18, wherein the tumor marker is methylated DNA.

20. The method according to any one of Claims 14 to 19, wherein the tumor marker is methylated DNA in the promoter region of a gene selected from the group consisting of SFRP1, SFRP2, DKK2, hsa-mir-34b/c, p16INK4A, E-cadherin, hMLH1, 14-3-3 sigma, BNIP3, CHFR, CIITA, IGFBP7, Histone H3K27, HRK, CACNA1G, COX2, DFNA5, and RASSF2.

21. The method according to Claim20, wherein the tumor marker is methylated DNA in the promoter region of a gene selected from the group consisting of SFRP1, SFRP2, DKK2, and hsa-mir-34b/c.

22. The method according to any one of Claims 14 to 21, wherein the index is a case wherein the methylated DNA in the promoter region of SFRP1 gene is more than 45%.

23. The method according to any one of Claims 14 to 21, wherein the index is a case wherein the methylated DNA in the promoter region of SFRP1 gene is more than 51%.

24. The method according to any one of Claims 14 to 21, wherein the index is a case wherein the methylated DNA in the promoter region of SFRP2 gene is more than 10%.

25. The method according to any one of Claims 14 to 21, wherein the index is a case wherein the methylated DNA in the promoter region of SFRP2 gene is more than 33%.

26. The method according to any one of Claims 14 to 21, wherein the index is a case wherein the methylated DNA in the promoter region of DKK2 gene is more than 10%.

27. The method according to any one of Claims 14 to 21, wherein the index is a case wherein the methylated DNA in the promoter region of DKK2 gene is more than 11%.

28. The method according to any one of Claims 14 to 21, wherein the index is a case wherein the methylated DNA in the promoter region of mir-34b/c gene is more than 15%.

29. The method according to anyone of Claims 14 to 21, wherein the index is a case wherein the methylated DNA in the promoter region of mir-34b/c gene is more than 18%.

30. The method according to any one of Claims 14 to 21, wherein the index is a case wherein the diameter of a tumor observed from the large intestinal lumen side is 20 mm or more and the methylated DNA in the promoter region of mir-34b/c gene is more than 15%.

31. The method according to any one of Claims 14 to 21, wherein the index is a case wherein the diameter of a tumor observed from the large intestinal lumen side is 20 mm or more and the methylated DNA in the promoter region of mir-34b/c gene is 15% or less, and the methylated DNA in the promoter region of DKK2 gene is more than 10%.

32. The method according to any one of Claims 14 to 21, wherein the index is a case wherein the diameter of a tumor observed from the large intestinal lumen side is 20 mm or more and the methylated DNA in the promoter region of SFRP1 gene is more than 51%, and the methylated DNA in the promoter region of SFRP2 gene is more than 10%.

33. The method according to any one of Claims 14 to 32, wherein the index is an index used for evaluating therapeutic effects of a drug and/or a therapeutic method.


SEQUENCE LISTING

<110>  Sapporo Medical University

<120>  Method to predict a cancer invasion utilizing colonic mucus

<130>  PCT413SI

<160>  14

<170>  PatentIn version 3.5

<210>  1
<211>  22
<212>  DNA
<213>  Artificial Sequence

<220>
<223>  Forward PCR primer for mir-34b/c


<220>
<221>  misc_feature
<223>  Y is either thymine or cytosine. For the primer to detect
       unmethylated cytosine in CpG island, Y means thymine. For the
       primer to detect methylated cytosine in CpG island, Y means
       cytosine.

<400>  1
ggtygagtga ttgtggyggg gg                                              22


<210>  2
<211>  26
<212>  DNA
<213>  Artificial Sequence

<220>
<223>  Reverse PCR primer for mir-34b/c


<220>
<221>  misc_feature
<223>  Y is either thymine or cytosine. For the primer to detect
       unmethylated cytosine in CpG island, Y means thymine. For the
       primer to detect methylated cytosine in CpG island, Y means
       cytosine.

<220>
<221>  misc_feature
<223>  R is either adenine or guanine. The resultant two primers having
       sequences of [cctccatctt ctaaacatct ccctta] or [cctccatctt
       ctaaacgtct ccctta] are used as a mixture.

<220>
<221>  misc_feature
<223>  For the reverse primer, 5' end is biotinylated.

<400>  2
cctccatctt ctaaacrtct ccctta                                          26


<210>  3
<211>  18
<212>  DNA

<213>  Artificial Sequence

<220>
<223>  Pyrosequencing primer for mir-34b/c


<220>
<221>  misc_feature
<223>  Y is either thymine or cytosine. For the primer to detect
       unmethylated cytosine in CpG island, Y means thymine. For the
       primer to detect methylated cytosine in CpG island, Y means
       cytosine.

<400>  3
taatygtttt tggaattt                                                    18


<210>  4
<211>  25
<212>  DNA
<213>  Artificial Sequence

<220>
<223>  Forward PCR primer for SFRP1


<220>
<221>  misc_feature
<223>  Y is either thymine or cytosine. For the primer to detect
       unmethylated cytosine in CpG island, Y means thymine. For the
       primer to detect methylated cytosine in CpG island, Y means
       cytosine.

<400>  4
gttttgtttt ttaaggggtg ttgag                                            25


<210>  5
<211>  26
<212>  DNA
<213>  Artificial Sequence

<220>
<223>  Reverse PCR primer for SFRP1


<220>
<221>  misc_feature
<223>  Y is either thymine or cytosine. For the primer to detect
       unmethylated cytosine in CpG island, Y means thymine. For the
       primer to detect methylated cytosine in CpG island, Y means
       cytosine.

<220>
<221>  misc_feature
<223>  R is either adenine or guanine. The resultant two primers having
       sequences of [ctccaaaaac tacaaaacta aaatac] or [ctccgaaaac
       tacaaaacta aaatac] are used as a mixture.

<220>
<221>  misc_feature
<223>  For the reverse primer, 5′ end is biotinylated.

<400>  5
ctccraaaac tacaaaacta aaatac                                           26

<210>  6
<211>  19
<212>  DNA
<213>  Artificial Sequence

<220>
<223>  Pyrosequencing primer for SFRP1


<220>
<221>  misc_feature
<223>  Y is either thymine or cytosine. For the primer to detect
       unmethylated cytosine in CpG island, Y means thymine. For the
       primer to detect methylated cytosine in CpG island, Y means
       cytosine.

<400>  6
gygtttggtt ttagtaaat                                                    19


<210>  7
<211>  15
<212>  DNA
<213>  Artificial Sequence

<220>
<223>  Pyrosequencing primer for SFRP1


<220>
<221>  misc_feature
<223>  Y is either thymine or cytosine. For the primer to detect
       unmethylated cytosine in CpG island, Y means thymine. For the
       primer to detect methylated cytosine in CpG island, Y means
       cytosine.

<400>  7
tygggagttg attgg                                                        15


<210>  8
<211>  26
<212>  DNA
<213>  Artificial Sequence

<220>
<223>  Forward PCR primer for SFRP2


<220>
<221>  misc_feature
<223>  Y is either thymine or cytosine. For the primer to detect
       unmethylated cytosine in CpG island, Y means thymine. For the
       primer to detect methylated cytosine in CpG island, Y means
       cytosine.

<400>  8
aatttyggat tggggtaaaa taagtt                                            26


<210>  9
<211>  27
<212>  DNA

<213>  Artificial Sequence

<220>
<223>  Reverse PCR primer for SFRP2


<220>
<221>  misc_feature
<223>  Y is either thymine or cytosine. For the primer to detect
       unmethylated cytosine in CpG island, Y means thymine. For the
       primer to detect methylated cytosine in CpG island, Y means
       cytosine.

<220>
<221>  misc_feature
<223>  For the reverse primer, 5′ end is biotinylated.

<400>  9
ttaaacaaca aacaaaaaaa cctaacc                                          27


<210>  10
<211>  19
<212>  DNA
<213>  Artificial Sequence

<220>
<223>  Pyrosequencing primer for SFRP2


<220>
<221>  misc_feature
<223>  Y is either thymine or cytosine. For the primer to detect
       unmethylated cytosine in CpG island, Y means thymine. For the
       primer to detect methylated cytosine in CpG island, Y means
       cytosine.

<400>  10
ygttttygtt agtatttgg                                                   19


<210>  11
<211>  25
<212>  DNA
<213>  Artificial Sequence

<220>
<223>  Forward PCR primer for DKK2


<220>
<221>  misc_feature
<223>  Y is either thymine or cytosine. For the primer to detect
       unmethylated cytosine in CpG island, Y means thymine. For the
       primer to detect methylated cytosine in CpG island, Y means
       cytosine.

<400>  11
aagtaaagag gattggggag agagt                                            25


<210>  12
<211>  26
<212>  DNA
<213>  Artificial Sequence

<220>
<223>  Reverse PCR primer for DKK2


<220>
<221>  misc_feature
<223>  Y is either thymine or cytosine. For the primer to detect
       unmethylated cytosine in CpG island, Y means thymine. For the
       primer to detect methylated cytosine in CpG island, Y means
       cytosine.

<220>
<221>  misc_feature
<223>  For the reverse primer, 5′ end is biotinylated.

<400>  12
ttatccccta actcacaaaa aacaac                                          26


<210>  13
<211>  20
<212>  DNA
<213>  Artificial Sequence

<220>
<223>  Pyrosequencing primer for DKK2


<220>
<221>  misc_feature
<223>  Y is either thymine or cytosine. For the primer to detect
       unmethylated cytosine in CpG island, Y means thymine. For the
       primer to detect methylated cytosine in CpG island, Y means
       cytosine.

<400>  13
gagagagtag agagagagaa                                                 20


<210>  14
<211>  20
<212>  DNA
<213>  Artificial Sequence

<220>
<223>  Pyrosequencing primer for DKK2


<220>
<221>  misc_feature
<223>  Y is either thymine or cytosine. For the primer to detect
       unmethylated cytosine in CpG island, Y means thymine. For the
       primer to detect methylated cytosine in CpG island, Y means
       cytosine.

<400>  14
taagttygtt ttttaggtat                                                 20